# EUROPÄISCHE PATENTSCHRIFT

(11) **EP 2 411 356 B1**
(45) Veröffentlichungstag und Bekanntmachung des Hinweises auf die Patenterteilung: **18.06.2014**
(21) Anmeldenummer: 10709794.1
(22) Anmeldetag: 03.03.2010
(51) Int. Cl.: C07C 51/235, C07C 53/122, C07C 53/124, C07C 53/126

(54) **VERFAHREN ZUR HERSTELLUNG ALIPHATISCHER CARBONSÄUREN AUS ALDEHYDEN DURCH MIKROREAKTIONSTECHNIK**
METHOD FOR PRODUCING ALIPHATIC CARBOXYLIC ACIDS FROM ALDEHYDES BY MICROREACTION TECHNOLOGY
PROCÉDÉ DE PRÉPARATION D'ACIDES CARBOXYLIQUES ALIPHATIQUES À PARTIR D'ALDÉHYDES PAR LA TECHNIQUE DE MICRORÉACTION

(30) Priorität: 24.03.2009 DE 102009014626
(43) Veröffentlichungstag der Anmeldung: 01.02.2012
(73) Patentinhaber: OXEA GmbH, 46147 Oberhausen (DE)
(72) Erfinder: WEBER, Tonia, 64293 Darmstadt (DE); KAUFMANN, Alexander, 46539 Dinslaken (DE); BORGMEIER, Oliver, 41470 Neuss (DE); KREICKMANN, Thorsten, 46149 Oberhausen (DE); STRUTZ, Heinz, 47445 Moers (DE); UTZ, Diana, 90763 Fürth (DE); KLEMM, Elias, 90411 Nürnberg (DE); LIEBOLD, Claudia, 09116 Chemnitz (DE)
(86) Internationale Anmeldenummer: PCT/EP2010/001298
(87) Internationale Veröffentlichungsnummer: WO 2010/108586

(56) Entgegenhaltungen:
- WO-A1-2004/108648
- WO-A2-2009/024549
- DE-C1- 10 010 770
- GB-A- 931 589
- EFRENFELD W ET AL: "Microreactors: New Technology for Modern Chemistry" 1. Januar 2000 (2000-01-01), 20000101, PAGE(S) 1 - 14 , XP002465177 ISBN: 978-3-527-29590-6 Abschnitte 1.2, 1.3
- UNBEKANNTER AUTOR: "Gezähmte Chemie im Mikroreaktor" VDI NACHRICHTEN, 2. Juni 2000 (2000-06-02) , XP002597026 Gefunden im Internet: URL:http://www.vdi-nachrichten.com/vdi-nac hrichten/aktuelle_ausgabe/akt_ausg_detail. asp?cat=2&id=2895&doPrint=1> [gefunden am 2010-08-18]

## Beschreibung

Die vorliegende Erfindung betrifft ein Verfahren zur Herstellung aliphatischer Carbonsäuren aus Aldehyden durch Oxidation mit Sauerstoff oder Sauerstoff enthaltenden Gasen durch Mikroreaktionstechnik bei erhöhtem Druck und bei einem Sauerstoffüberschuss.

Aldehyde sind die gebräuchlichen Ausgangsstoffe zur Gewinnung von Carbonsäuren. Die Vorzugsstellung für dieses Einsatzgebiet verdanken sie ihrer mannigfachen Verfügbarkeit und der oxidativen Umwandlung der Carbonylgruppe in die Carboxylgruppe. Im Rahmen technisch ausgeübter Verfahren erfolgt die Umsetzung von Aldehyden zu Carbonsäuren vorwiegend in Anwesenheit von Katalysatoren. Es sind jedoch auch Prozesse bekannt, bei denen auf die Verwendung von Katalysatoren verzichtet wird. Zur Vermeidung von Nebenreaktionen arbeitet man sowohl bei den katalytischen als auch bei den nicht katalytischen Prozessen bei möglichst niedrigen Temperaturen, im Allgemeinen wird eine Reaktionstemperatur von 100°C nicht überschritten. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Häufig ist die Carbonsäurebildung aus Aldehyden, selbst bei Einhaltung optimaler Temperaturbedingungen, mit Neben- und Abbaureaktionen verbunden. Das gilt gleichermaßen für Reaktionen in Gegenwart wie in Abwesenheit von Katalysatoren. In solchen Fällen kann die Selektivität der Umsetzung durch Zusatz von Alkalimetallsalzen schwacher Säuren zu den Reaktanden erheblich verbessert werden. Nachteilig bei dieser Verfahrensvariante ist jedoch, dass die Salze inhibierend wirken, so dass für eine vollständige Umsetzung der Ausgangsstoffe die Reaktionszeit verlängert werden muss. Die Oxidation aliphatischer Aldehyde zu den entsprechenden Carbonsäuren unter Verwendung von Sauerstoff ist ein seit vielen Jahren großtechnisch betriebenes Verfahren. Wichtige aliphatisches Carbonsäuren, die nach diesem Verfahren gestellt werden, sind die isomeren Buttersäuren, die isomeren Pentansäuren, 2-Ethylhexansäure, n-Heptansäure, n-Nonansäure sowie iso-Nonansäure auf Basis von 3,5,5-Trimethylhexansäure (Ullmann's Encyclopedia of Industrial Chemistry, Wiley-VCH, 6. Auflage, Band 6, Kapitel "Carboxylic Acids, Aliphatic"; K.Weissermel, H.-J. Arpe, Industrielle Organische Chemie, VCH Verlagsgesellschaft, 3. Auflage, 1988, Kapitel "Oxo-Carbonsäuren").

Nach einer konventionellen Ausführungsform der Oxidation aliphatischer Aldehyde legt man den Aldehyd in einem geeigneten Reaktor, z.B. in einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd. Eine derartige Verfahrensweise unter Verwendung einer Blasensäule wird beispielsweise in der DE 100 107 69 C1, DE 100 107 70 C1 und DE 100 107 71 C1 beschrieben. Die Umsetzung des flüssigen, aliphatischen Aldehyds mit gasförmigem Sauerstoff wird im Allgemeinen bei Temperaturen von vorzugsweise 40 bis 80°C und bei Normaldruck durchgeführt. Höhere Reaktionstemperaturen und Drücke sind zwar möglich, doch sollte die gewählte Reaktionstemperatur 100°C nicht überschreiten und der Druck nicht höher als 1 MPa sein. Bekannt sind sowohl nichtkatalytische Verfahren (DE 100 107 69 C1, DE 100 107 69 C1) als auch übergangsmetallkatalysierte Verfahren (DE 100 107 71 C1; G.R. Lappin, J.G. Sauer "Alpha Olefins Applications Handbook", Marcel Dekker Inc., New York and Basel, 1989, Kapitel 11, "Fatty Acids"). Entsprechend einer weiteren Ausführungsform verwendet man als Reaktor einen Rieselturm, der Füllkörper enthält. Über die Füllung lässt man den Aldehyd herabrieseln und leitet in den Turm gleichzeitig im Gleich- oder Gegenstrom Sauerstoff oder ein Sauerstoff enthaltendes Gasgemisch ein. Daneben wird bei der Aldehydoxidation mit gasförmigem Sauerstoff auch der Einsatz von Rührkesseln im Stand der Technik beschrieben (WO2001/46111 A1).

W02009/024549 A2 behandelt ein Oxidationsverfahren von organischen Stoffen mit Sauerstoff in einer ersten Reaktionsstufe und die anschließende Zugabe des Reaktionsgemisches aus der ersten Reaktionsstufe in eine weitere, adiabat betriebene Reaktionsstufe. Die erste Reaktionsstufe kann auch in verschiedene Reaktionsstufen gegliedert werden, in denen sauerstoffhaltiges Gas eingeleitet werden kann. Das bekannte Verfahren eignet sich zur Oxidation von Aldehyden zu den entsprechenden Carbonsäuren.

Die technische Oxidation von aliphatischen Aldehyden mit gasförmigem Sauerstoff in großvolumigen, konventionellen Reaktoren wird stets optimiert und mittlerweile bei hohem Umsatz mit guten Carbonsäureselektivitäten betrieben. Nachteilig sind jedoch die vergleichsweise langen Verweilzeiten des Reaktionsgutes, die bis zu mehreren Stunden betragen können und die Wirtschaftlichkeit des Oxidationsprozesses beeinträchtigen. Bei langen Verweilzeiten in den Oxidationsreaktoren ist zusätzlich mit einer verstärkten Nebenproduktbildung zu rechnen, beispielsweise mit Spaltprodukten wie niedrigere Carbonsäuren, Kohlenmonoxid oder Kohlendioxid, so dass die Selektivität der Oxidationsreaktion trotz der mittlerweile erreichten guten Werte leiden kann. Ebenfalls erfordert der Umgang von aliphatischen Aldehyden und Sauerstoff in großvolumigen Reaktoren einen hohen Investitions- und Kontrollaufwand für sicherheitstechnische Einrichtungen. Auch muss aufgrund der exothermen Oxidationsreaktion dafür Sorge getragen werden, dass die freigesetzte Reaktionswärme rasch und kontrolliert abgeführt werden kann, um einen unerwünschten Temperaturanstieg zu vermeiden. Daher sind entsprechend dimensionierte Kühleinrichtungen zu installieren. Für eine Erniedrigung der Verweilzeit kann zwar das Arbeiten bei erhöhten Drücken ins Auge gefasst werden, doch ihre Anwendung bei der Sauerstoffoxidation von aliphatischen Aldehyden in großvolumigen Reaktoren kann aus sicherheitstechnischen Gründen sehr problematisch werden und erfordert einen hohen Investitionsbedarf. Technisch betriebene Aldehydoxidationen werden daher meist bei Normaldruck oder gegebenenfalls nur leichtem Überdruck durchgeführt.

Die in der Informations- und Kommunikationstechnologie seit den 1960er stetig fortschreitende Miniaturisierung hat seit den 1980er auch auf dem Gebiet der Chemie zunächst im Labor und in den letzten Jahren auch zunehmend in der Produktion Einzug gehalten. Während bei den sogenannten Hochdurchsatzuntersuchungen die Miniaturisierung von Reaktionsräumen dazu genutzt wird, eine bestimmte Reaktion auf kleinem Raum in paralleler Weise unter möglichst vielen verschiedenen Bedingungen auszutesten, um beispielsweise geeignete Katalysatoren gezielt zu entwickeln (WO 00/51720), dient die Miniaturisierung in der Mikroreaktionstechnik der Durchführung einer chemischen Reaktion unter denselben Bedingungen in einer Vielzahl paralleler Reaktionsräume, um entsprechende Mengen chemischer Produkte unter definierten Bedingungen herstellen zu können. Der Übergang von der makroskopischen Verfahrensführung auf die Mikroreaktionstechnik bietet folgende verfahrenstechnische Vorteile: In Folge der kleinen lateralen Abmessungen werden Wärme- und Stofftransportvorgänge durch Wärmeleitung und Diffusion stark intensiviert und ermöglichen hohe Leistungen für Wärmeüberträger und Mischer. Bei exothermen Reaktionen kann daher die Reaktionswärme wesentlich schneller abgeführt werden als in großvolumigen, konventionellen Reaktoren. Charakteristisch für die Mikroreaktionstechnik ist das hohe Verhältnis von Oberfläche zu Volumen, also die hohe spezifische Oberfläche, so dass auch oberflächenbestimmte Phänomene deutlich intensiviert werden können.

Als Folge dieser Intensivierungen sind höhere Raum-Zeit-Ausbeuten, d.h. kürzere Verweilzeiten, möglich. Das Intensivierungspotential der Mikroreaktoren wird insbesondere dann voll ausgeschöpft, wenn neue Reaktionsbedingungen eingestellt werden, die für konventionelle Reaktoren unüblich sind oder in solchen konventionellen Reaktoren nicht mehr realisiert werden können. Man spricht in diesem Zusammenhang von ""novel process windows", ein Begriff, der beispielsweise aus Energy Environ. Sci., 2008, 1, Seiten 467-478, bekannt ist. Insbesondere die Einstellung höherer Drücke und Temperaturen ist dadurch möglich. Hinzu kommt der sicherheitstechnische Aspekt, dass diese Bedingungen in großvolumigen, konventionellen Reaktoren häufig nicht mehr kontrolliert werden können und nur in Mikroreaktoren sicher eingestellt werden können. Aber auch ohne dieses Intensivierungspotential, das heißt ohne die Anwendung ungewöhnlich hoher Drücke und Temperaturen, kann der Mikroreaktor einen sichereren Betrieb als mit konventioneller Technologie ermöglichen.

Typisch für die Mikroreaktionstechnik sind Apparatedimensionen in einem Bereich von einigen Millimetern bis einigen Mikrometern. Für die Fertigung mikroverfahrenstechnischer Bauteile in diesen Dimensionen stehen geeignete Strukturierungsmethoden zur Verfügung, beispielsweise feinwerktechnische Verfahren wie Mikrozerspannung oder Mikrofunkenerosion, Laserablationsverfahren, Umform- und Abformverfahren oder Ätzverfahren. Bei Abmessungen insbesondere unterhalb von 100 Nanometern spricht man von der Nanotechnologie während man bei Apparateabmessungen oberhalb von einigen Millimetern zunächst Milliapparate versteht. Dann schließen sich konventionelle Reaktoren wie Rohrbündelapparate, Rohrreaktoren und schließlich Rührkessel an.

Eine Übersicht über das Gebiet der Mikroverfahrenstechnik und über die Fertigung mikroverfahrenstechnischer Komponenten findet sich im Winnacker, Küchler, Chemische Technik, Wiley-VCH 5. Auflage, 2004, Band 2, Kapitel 8 "Mikroverfahrenstechnik" und bei Microreactors in Organic Synthesis and Catalyst, Wiley-VCH, 2008, Kapitel 1 "Fabrication of Microreactors Made from Metals and Ceramics" und Kapitel 2 "Fabrication and Assembling of Microreactors Made from Glass and Silicon".

Vor dem Hintergrund der zuvor geschilderten Nachteile, die die Oxidation von aliphatischen Aldehyden mit Sauerstoff oder Sauerstoff enthaltenden Gasen in konventionellen, großvolumigen Reaktoren aufweist, ist die Aufgabe der vorliegenden Erfindung, ein Verfahren zur Oxidation von aliphatischen Aldehyden zu den entsprechenden Carbonsäuren bereitzustellen, das bei hohem Aldehydumsatz und hohen Selektivitäten zu den gewünschten Carbonsäuren eine Erniedrigung der Verweilzeit ermöglicht. Die damit einhergehende Erhöhung der Raum-Zeit-Ausbeute an der gewünschten Carbonsäure erhöht die Wirtschaftlichkeit des Oxidationsprozesses. Auch soll das bereitzustellende Verfahren sicherer betrieben werden und sich durch einen geringeren Investitionsbedart für Sicherheitseinrichtungen auszeichnen.

Die vorliegende Erfindung betrifft daher ein Verfahren zur Herstellung aliphatischer Carbonsäuren mit 3 bis 10 Kohlenstoffatomen durch Oxidation der entsprechenden Aldehyde mit Sauerstoff oder Sauerstoff enthaltenden Gasen. Es ist dadurch gekennzeichnet, dass die Reaktion in einem Mikroreaktor bei erhöhtem Druck und bei einem Sauerstoffüberschuss, bezogen auf die stöchiometrisch erforderliche Sauerstoffmenge, durchgeführt wird und dass das aus dem Mikroreaktor abgeführte Reaktionsgemisch ohne weitere Sauerstoffzugabe durch mindestens einen Nachreaktor geleitet wird und wobei die Oxidationsreaktion in dem Mikroreaktor unter solchen Bedingungen durchgeführt wird, dass im abgeführten Reaktionsgemisch Aldehyd und gebildete entsprechende Percarbonsäure in einem molaren Verhältnis in einem Bereich von 1 : (0,9 bis 1,1), vorzugsweise von 1 : (0,95 bis 1,05) vorliegen.

Bei dem mittels Mikroreaktionstechnik erfindungsgemäß durchgeführten Oxidationsverfahren wird gasförmiger Sauerstoff oder ein Sauerstoff enthaltendes Gas mit dem flüssigen, aliphatischen Aldehyd in Kontakt gebracht. Daher werden sich in dem Mikroreaktor, beispielsweise in einem Mikrokapillarreaktor mit Mikrokanälen, Gas/Flüssig-Mehrphasenströmungen ausbilden, die je nach Strömungsgeschwindigkeit der gasförmigen und flüssigen Phase bestimmte Strömungscharakteristiken zeigen. Man unterscheidet hier zwischen einer Blasenströmung, bei der die Gasphase dispers vorliegt und die Gasblasen nahezu kugelförmig sind und ihre Durchmesser kleiner sind als der Durchmesser des Mikrokanals, einer Pfropfenströmung, bei der die Gasphase weiterhin dispers vorliegt, die Gasblasen aber eine zylindrische Form unterschiedlicher Länge haben und den gesamten Querschnitt des Mikrokanals ausfüllen, wobei es zu einer Segmentierung in alternierende Gas- und Flüssigkeitspfropfen kommt, und einer Ringströmung, bei der die Gasphase eine kontinuierliche Kernströmung mit einem Flüssigkeitsfilm an der Mikrokanalwandung bildet. Auf die Strömungscharakteristik von Gas-Flüssig-Mehrphasenströmungen wird im Winnacker, Küchler, Chemische Technik, Wiley-VCH 5. Auflage, 2004, Band 2, Kapitel 3 "Mikrofluidik" eingegangen.

Für die Aldehydoxidation mit Sauerstoff oder Sauerstoff enthaltenden Gasen nach dem erfindungsgemäßen Verfahren eignen sich sämtliche Mikroreaktoren, in denen sich bei der Umsetzung der gasförmigen sauerstoffhaltigen Phase mit der flüssigen aldehydhaltigen Phase eine heterogene Gas/Flüssigströmung ausbildet. Die sich ausbildende Strömungscharakteristik der Blasen-, Pfropfen- oder Ringströmung oder ihrer Mischungen stellt eine ausreichende Durchmischung der gasförmigen mit der flüssigen Phase sicher. Beispiele für Mikroreaktoren, die sich für die Durchführung des erfindungsgemäßen Verfahrens eignen, sind aus Chemical Micro Process Engineering. Wiley-VCH, 2004, Kapitel 5.1 "Micro Reactors for Gas/Liquid Reactions" bekannt. Besonders geeignete Mikroreaktoren sind Mikrokapillarreaktoren oder Mikroblasensäulenreaktoren mit entsprechend dimensionierten Mikrokanälen, in denen sich die zuvor genannten Strömungscharakteristiken ausbilden können. Solche Mikroreaktoren werden beispielsweise in Microreactors in Organic Synthesis and Catalyst, Wiley-VCH, 2008, Kapitel 4.4 "Gas-Liquid-Reactions" oder in Fortschritts-Berichte VDI, Reihe 3 Nr. 771, VDI Verlag GmbH, Düsseldorf 2003 beschrieben. Ebenfalls können eine Vielzahl von Mikroreaktoren mit entsprechend dimensionierten Mikrokanälen in einem Mehrkanalsystem parallel angeordnet werden.

Weiterhin ist es möglich, vor dem eigentlichen Mikroreaktor, beispielsweise vor dem Mikrokapillarreaktor, einen Mikromischer zu betreiben und anschließend die Reaktionsmischung dem Mikroreaktor zuzuführen. Geeignete Mischertypen sind beispielsweise aus Winnacker, Küchler, Chemische Technik, Wiley-VCH 5. Auflage, 2004, Band 2, Kapitel 4.2 "Mischen" oder bei Chemical Micro Process Engineering, Wiley-VCH, 2004, Kapitel 4.1 "Micro Reactors for Liquid-phase and Liquid/Liquid-phase Reactions" bekannt. Diese zunächst für die intensive Vermischung von Flüssigkeiten konzipierten Mischertypen sind ebenfalls für das Vermischen einer gasförmigen Phase mit der flüssigen Phase geeignet.

Bei solchen Mikromischern werden die flüssige Phase und die gasförmige Phase über kapillare Zuleitungen in den Mischer eingeleitet, in einer Mischkammer vermischt und aus der Mischkammer abgeleitet. Es ist auch möglich, dass die beiden kapillaren Zuleitungen in einem Bereich münden, der Ausgangspunkt einer kapillaren Ableitung ist, in der das heterogene Gas/Flüssiggemisch dem Mikroreaktor zugeführt wird. Wenn die kapillaren Zuleitungen in einem Winkel von etwa 180° angeordnet sind und so die gasförmige und flüssige Phase frontal aufeinandertreffen und die kapillare Ableitung in einem Winkel von etwa 90° zu dem Mündungsbereich der kapillaren Zuleitungen angeordnet ist, erhält man eine T-förmige Mischeinrichtung. Daneben können die beiden kapillaren Zuleitungen auch in einem Winkel zwischen 90 und 180° oder in einem Winkel zwischen 0 und 90° angeordnet sein und zusammen mit der kapillaren Ableitung eine Y-förmige Mischeinrichtung bilden.

Als besonders geeignet haben sich Mikrokapillarreaktoren erwiesen. Bei Mikrokapillarreaktoren kann es sich um eine einzelne Mikrokapillare handeln, der vorzugsweise noch ein Mikromischer vorgeschaltet ist. Daneben eignen sich auch solche Mikrokapillarreaktoren, bei denen eine Platte mit Mikroriefen versehen ist, die eine Vielzahl von parallelen Mikroreaktionskanälen bilden. Eine derartige Anordnung von parallelen Mikroreaktionskanälen kann auch als Mikroblasensäulenreaktor betrieben werden, bei dem die flüssige Phase auf dem Kopfbereich der senkrecht angeordneten Platte gegeben wird und der Flüssigkeitsfilm in Richtung Boden fällt. Die gasförmige Phase kann im Gleichstrom, Gegenstrom oder Kreuzstrom zugeführt werden. Aus DE 102 57 239 B3 ist ein geeigneter Mikroblasensäulenreaktor bekannt, in dem eine flüssige, olefinhaltige Lösung mit gasförmigem Sauerstoff in Gegenwart eines Photosensibilisators in einem heterogenen Reaktionssystem oxidiert wird.

WO 2007/027785 A2 beschreibt die Umsetzung einer wasserstoffhaltigen organischen Verbindung, beispielsweise eines Hydrochinonderivats, mit gasförmigem Sauerstoff in einem Mikroreaktor unter Bildung des Zielprodukts Wasserstoffperoxid. Als Mikroreaktor kann beispielsweise eine parallele Anordnung von einer Vielzahl von Mikroreaktionskanälen verwendet werden.

DE 10 2004 049 730 A1 offenbart die katalytische Oxidation von organischen Verbindungen unter Verwendung eines Mikrokapillarreaktors enthaltend mindestens zwei statische Mischer, wobei in dem ersten Mischer zwei nicht miteinander homogen mischbare Flüssigkeiten zusammengeführt werden und die so erhaltene flüssige heterogene Phase in einen nachgeschaltenen zweiten Mischer geleitet wird, in dem über eine separate kapillare Leitung eine gasförmige Komponente eingeführt wird. Das resultierende flüssig/flüssig/gasförmige Gemisch wird anschließend durch eine Kapillare geleitet. Bei der einen flüssigen Phase handelt es sich um eine organische Phase mit mindestens einem darin gelösten organischen und mittels Sauerstoff oxidierbaren Edukt und bei der anderen flüssigen Phase um eine wässrige, den gelösten Oxidationskatalysator enthaltende Lösung. Sauerstoff wird als gasförmige Phase in den zweiten Mischer geleitet.

Die Oxidation von n-Butyraldehyd mittels gasförmigem Sauerstoff in einer Mikroblasensäule wird bei Microreaction Technology, IMRET: Proceedings of the 5th. International Conference on Microreaction Technology; Springer-Verlag, 2001, Seiten 202-214 und bei Fortschritts-Berichte VDI Reihe 3 Nr. 771, VDI Verlag GmbH, Düsseldorf 2003; Seiten 108-125 und 143-144 behandelt. Das Umsatzverhalten in der Mikroblasensäule wird entscheidend durch das Zusammenwirken zwischen der spezifischen Oberfläche und der Verweilzeit beeinflusst und durch den Vergleich der experimentell gefundenen Umsatzdaten mit einer für die Mikroblasensäule durchgeführten Modellrechnung kann bei einem gegebenen Flüssigkeitsvolumenstrom ein optimaler n-Butyraldehydumsatz bei einer bestimmten spezifischen Oberfläche und Verweilzeit berechnet werden. Die berichteten Umsatzzahlen liegen im Bereich um 40%, bezogen auf eingesetzten Aldehyd.

Es hat sich bewährt, die Aldehyde gemäß dem neuen Verfahren bei Temperaturen zwischen 0 und 130°C, vorzugsweise zwischen 20 und 100°C und insbesondere zwischen 40 und 80°C zu oxidieren. Die Umsetzung wird bei einer konstanten oder, innerhalb der technischen Möglichkeiten, annähernd konstanten Temperatur vorgenommen. Für das Einhalten der Temperaturkonstanz kann der Mikroreaktor in einem Gefäß mit einem Wärmeträgermedium angeordnet sein. Durch Umpumpen des Wärmeträgermediums wird kontinuierlich Wärme abgeführt oder zugeführt. Daneben können an den Mikroreaktoren ebenfalls Mikrowärmeaustauscherplatten angebracht sein, durch die das Wärmeträgermedium fließt und die Temperaturkonstanz in dem Mikroreaktor sicherstellt.

Das aus dem Mikroreaktor abgeführte Reaktionsgemisch wird zunächst in einem Druckabscheider auf Normaldruck entspannt, wobei sich eine flüssige Phase abscheidet und Abgas gebildet wird. Das sauerstoffhaltige Abgas wird ausgeschleust und die flüssige Phase zur weiteren Aufarbeitung abgeführt.

Nach dem erfindungsgemäßen Verfahren wird das in dem Druckabscheider erhaltene flüssige Reaktionsprodukt aus der ersten Reaktionsstufe einer zweiten Reaktionsstufe zugeführt, die ebenfalls in einem Mikroreaktor erfolgt. Dabei kann der in der zweiten Reaktionsstufe verwendete Mikroreaktor mit dem in der ersten Stufe eingesetzten Mikroreaktor baugleich sein, es kann aber auch ein davon baulich abweichender, üblicher Mikroreaktor verwendet werden.

Die abgeschiedene flüssige Phase aus der ersten Reaktionsstufe wird nach dem Entspannungsschritt ohne weitere Zugabe von Sauerstoff in den zweiten Mikroreaktor gepumpt, wobei sich durch den Pumpvorgang ein Flüssigkeitsdruck ausbildet. Man kann daher den ersten Mikroreaktor als den Oxidationsreaktor und den zweiten Mikroreaktor als einen Nachreaktor auffassen. Da dem zweiten Mikroreaktor keine gasförmige Phase zugespeist wird, können für diesen Prozessschritt auch Mikroreaktoren eingesetzt werden, die nicht unbedingt einen guten Stoffaustausch zwischen einer gasförmigen und flüssigen Phase gewährleisten müssen. Der zweite Mikroreaktor oder Nachreaktor wird durch geeignete Wärmeaustauscheraggregate auf eine Temperatur eingestellt, die der Temperatur in dem ersten Mikroreaktor entspricht. Es ist auch möglich, den Nachreaktor bei einer tieferen Temperatur zu betreiben, wobei die Temperaturdifferenz 5 bis 50°C, vorzugsweise 10 bis 20°C beträgt. Vorzugsweise werden bei dieser zweistufigen Arbeitsweise Mikroreaktor und Nachreaktor bei der gleichen Temperatur betrieben.

Gegebenfalls kann das nach dem zweiten Mikroreaktor anfallende Reaktionsgemisch nochmals durch einen dritten oder durch weitere Mikroreaktoren ohne Zugabe von Sauerstoff gepumpt werden. Bei dieser Ausführungsform des erfindungsgemäßen Verfahrens erfolgt somit in dem ersten Mikroreaktor die Umsetzung bei erhöhtem Druck und bei einem Sauerstoffüberschuss, bezogen auf die stöchiometrisch erforderliche Sauerstoffmenge, während nach dem Entspannungsschritt in einer Kaskade von nachgeschalteten Mikroreaktoren oder Nachreaktoren ohne aktive Zugabe von Sauerstoff die Reaktion beendet wird. Die Mikroreaktoren in der Nachreaktionskaskade können bei der Temperatur des ersten Mikroreaktors betrieben werden. Ebenfalls ist es möglich, innerhalb der Nachreaktorkaskade zwischen benachbarten Stufen eine Temperaturdifferenz einzustellen, wobei von Stufe zu Stufe die Temperatur abnimmt. Die Temperaturdifferenz kann zwischen 5 und 50°C, vorzugsweise 10 bis 20°C, betragen. Die genannten Temperaturangaben stellen lediglich Richtwerte dar, die im Einzelfall auf die speziellen Gegebenheiten abzustimmen sind. Werden mehr als zwei Reaktionsstufen, also die Umsetzung unter Sauerstoffüberdruck in dem ersten Mikroreaktor und eine Nachreaktionsstufe, angewandt, ist es nicht erforderlich, zwischen den einzelnen Stufen gleiche Temperaturunterschiede einzuhalten. Die Temperaturdifferenzen können vielmehr variabel gewählt und den individuellen Anforderungen angeglichen werden. Das aus dem zweiten oder bei mehreren Nachreaktoren aus dem letzten Mikroreaktor abfließende Rohsäuregemisch wird in einer Vorlage gesammelt und kann anschließend nach bekannten Destillationsverfahren zur Reinware aufdestilliert werden.

Überraschenderweise kann bei der zweistufigen oder mehrstufigen Verfahrensführung die Carbonsäureselektivität gegenüber der einstufigen Fahrweise nochmals verbessert werden, wenn die Oxidationsreaktion in dem ersten Mikroreaktor bei erhöhtem Druck und bei einem Sauerstoffüberschuss, bezogen auf die stöchiometrisch erforderliche Menge, unter solchen Bedingungen durchgeführt wird, dass im Reaktionsaustrag Aldehyd und gebildete Percarbonsäure im molaren Verhältnis in einem Bereich von 1 : (0,9 bis 1,1), vorzugsweise von 1 : (0,95 bis 1,05), vorliegen. Ohne auf genaue mechanistische Überlegungen einzugehen, kann angenommen werden, dass unter diesen Reaktionsbedingungen der Aldehydumsatz in der ersten Reaktionsstufe noch nicht vollständig ist und in dem Reaktionsaustrag Percarbonsäure und Aldehyd etwa im gleichen molaren Verhältnis vorliegen, die dann in dem Nachreaktor nach einer Baeyer-Villiger-Reaktion zur gewünschten Carbonsäure kontrolliert abreagieren. Nach der zwei- oder mehrstufigen Fahrweise kann die unkontrollierte Zersetzung der Percarbonsäure, die die Selektivität der Umsetzung beeinträchtigt, wirksam unterbunden werden. Bei dieser Art der Reaktionsführung wird somit in der ersten, bei erhöhtem Druck und bei Sauerstoffüberschuss durchgeführten Reaktionsstufe nur auf einen Teilumsatz an Aldehyd hin gearbeitet. Die Restmengen an Aldehyd werden dann in der Nachreaktion durch die Umsetzung mit der Percarbonsäure verbraucht. Bei der zweistufigen oder mehrstufigen Fahrweise wird vorzugsweise die Oxidationsreaktion bei einem Druck von 0,4 bis 2,0 MPa und bei einem Sauerstoffüberschuss, der der bis zur zweifachen erforderlichen Sauerstoffmenge entspricht, durchgeführt und das erhaltene Reaktionsgemisch ohne weitere aktive Zugabe von Sauerstoff durch einen zweiten oder mehrere nachgeschaltete Nachreaktoren, die ebenfalls als Mikroreaktoren konzipiert sind, geleitet.

Durch den Einsatz von Mikroreaktoren nicht nur in der ersten, unter Sauerstoffzugabe durchgeführten Oxidationsreaktion sondern auch in der folgenden Nachreaktionsstufe kann die Verweilzeit gegenüber einem konventionell in großvolumigen Reaktoren durchgeführten Aldehydoxidationsprozess ebenfalls deutlich reduziert werden. So beträgt die Verweilzeit je Reaktorstufe 0,5 bis 10 Minuten, vorzugsweise 1 bis 5 Minuten. Gleichzeitig werden hervorragende Aldehydumsätze und Carbonsäureselektivitäten in dem aus dem letzten Nachreaktor austretenden Rohsäuregemisch beobachtet.

Unabhängig davon, ob die Oxidationsreaktion in einem Mikroreaktor in Kombination mit einem oder mehreren Nachreaktoren, von denen mindestens ein Nachreaktor als Mikroreaktor konzipiert ist, durchgeführt wird, besitzen die verwendeten Mikroreaktoren im Allgemeinen einen Reaktionsraum mit einem Durchmesser von 1 Millimeter bis 0,01 Millimetern.

Im Mittelpunkt des neuen Prozesses steht die Oxidation von unverzweigten und verzweigten C₃- bis C₁₀-Aldehyden. Die Herkunft der Aldehyde ist nicht auf bestimmte Herstellungsverfahren beschränkt. Aufgrund ihrer einfachen Zugänglichkeit werden durch Oxosynthese, d.h. durch Reaktion von C₂- bis C₉-Olefinen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Besonders eignet sich das erfindungsgemäße Verfahren zur Herstellung von Propionsäure, n-Buttersäure, iso-Buttersäure, n-Pentansäure, 2-Methylbuttersäure, 3-Methylbuttersäure, n-Hexansäure, 2-Methylpentansäure, n-Heptansäure, 2-Methylhexansäure, 2-Ethylhexansäure, 2-Propylheptansäure, n-Nonansäure, 2-Methyloctansäure sowie iso-Nonansäure mit dem Hauptbestandteil 3,5,5-Trimethylhexansäure, wobei der entsprechende Aldehyd durch Oxo-Synthese von Di-isobutylen zugänglich ist.

Als Oxidationsmittel verwendet man nach dem Verfahren der Erfindung molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten, Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Das erfindungsgemäße Verfahren wird im Allgemeinen kontinuierlich durchgeführt. Eine Rückführung nicht umgesetzter Reaktionsteilnehmer ist möglich.

Um die Menge an Reaktionsprodukt aus der Aldehydoxidation pro Zeiteinheit zu erhöhen, können eine Vielzahl von Mikrokanälen in einem Mehrkanalsystem sowie Mikroreaktoren, beispielsweise Mikroblasensäulen, parallel betrieben werden. Auf diese Weise erhöht man die Produktionsmenge und kann gleichzeitig die Vorteile der Mikroreaktionstechnik, wie hohe spezifische Oberfläche und gute Wärmeabfuhr nutzen.

In den folgenden Beispielen wird die Herstellung von n-Pentansäure und iso-Nonansäure nach dem beanspruchten Verfahren beschrieben. Selbstverständlich ist das neue Verfahren nicht auf die nachstehend beschriebenen Ausführungsformen beschränkt.

### Beispiele

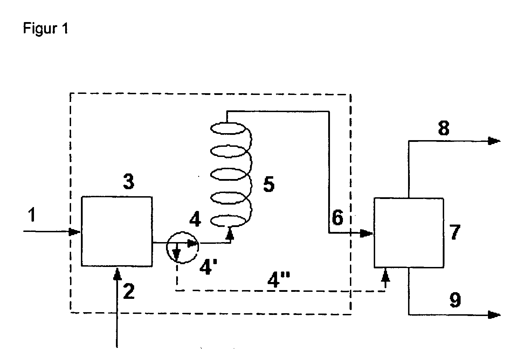

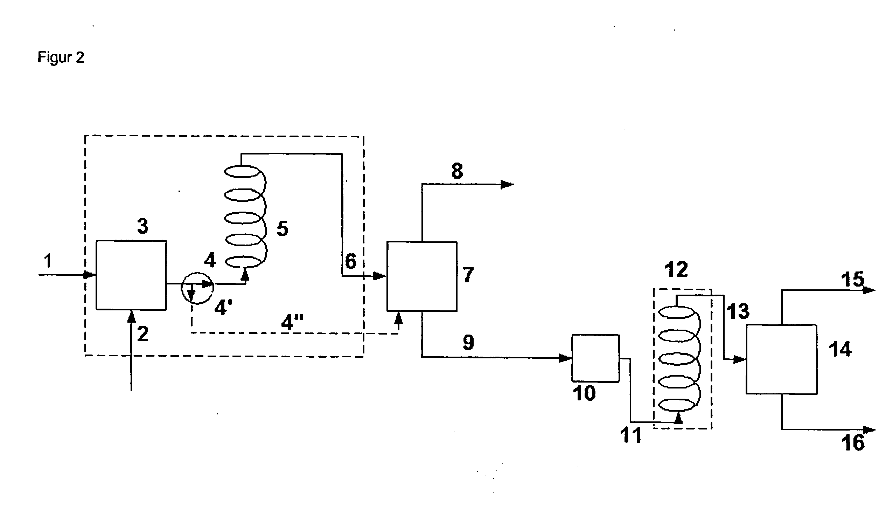

Die Flüssigphasenoxidation der eingesetzten Aldehyde wird gemäß folgendem Versuchsaufbau durchgeführt, der in Figur 1 und 2 prinzipiell dargestellt ist. Über die Leitung (1) wird flüssiger Aldehyd und über die Leitung (2) gasförmiger Sauerstoff dem Mikromischer (3), bei dem es sich um ein Mikro-T-Stück mit einem Innendurchmesser von 0,75 mm handelt, zugeführt. Das Eduktgemisch wird anschließend über die Leitung (4), die ebenfalls einen Innendurchmesser von 0,75 mm besitzt, einem Dreiwegehahn (4') zugeleitet. Für die Analytik des Eduktstroms wird der Dreiwegehahn (4') auf eine Art By-Pass-Modus eingestellt, bei dem der Eduktstrom unter Umgehen des Mikroreaktors (5) über die Leitung (4") direkt in einen Druckabscheider (7) fließt, in dem er entspannt wird. Die dabei erhaltene flüssige Phase wird gaschromatographisch analysiert. Im Arbeitsbetrieb wird der Dreiwegehahn (4') so eingestellt, dass der Eduktstrom in den Mikroreaktor (5) fließt. Der Mikroreaktor (5) besteht aus einer 5 m langen Mikrokapillare mit einem Innendurchmesser von 0,75 mm. Das Eduktgemisch tritt von unten in den Mikroreaktor (5) ein und an seinem Kopf wird das Produktgemisch über die Leitung (6) abgeführt und dem Druckabscheider (7) zugeführt, in dem das Produktgemisch auf Normaldruck entspannt wird. Sauerstoffhaltiges Abgas wird über die Leitung (8) ausgeschleust während die angefallene Rohsäure über die Leitung (9) entnommen wird und nach konventionellen Verfahren destillativ aufgereinigt wird. Der Mikroreaktor (5) und der Mikromischer (3) befinden sich in einer Temperierzelle, die schematisch angedeutet ist. Die Temperierzelle wird über einen Thermostaten geheizt.

Figur 2 beschreibt schematisch die erfindungsgemäße, zweistufige Reaktionsführung. Die Bezugszeichen (1)-(9) haben die zuvor genannte Bedeutung. Das aus dem Druckabscheider (7) der ersten Reaktionsstufe über Leitung (9) herangeführte flüssige Rohgemisch wird mittels der Kolbenpumpe (10) über die Leitung (11) mit einem Innendurchmesser von 0,75 mm dem zweiten Mikroreaktor (12) oder Nachreaktor von unten zugeführt. Bei dem Mikroreaktor (12) handelt es sich ebenfalls um eine Mikrokapillare mit einer Länge von 5 m bei einem Innendurchmesser von 0,75 mm. Das Rohprodukt wird am Kopf des zweiten Mikroreaktors abgenommen und über die Leitung (13) in eine Vorlage (14) geführt. Gasförmige Anteile werden über die Leitung (15) entnommen und über die Leitung (16) wird die Rohsäure in die destillative Aufarbeitung gegeben. Der zweite Mikroreaktor befindet sich ebenfalls in einer Temperiervorrichtung, die schematisch angedeutet ist.

Über die Leitung (1) wird der jeweilige Aldehyd in einer Menge von 0,9 ml/min mittels einer Kolbenpumpe und über Leitung (2) Sauerstoff aus einer Druckflasche in einer Menge von 140 ml/min dem Mikromischer (3) zugeführt. Das molare Verhältnis von Sauerstoff zu Aldehyd beträgt 1,5, die Verweilzeit des Aldehyds im ersten Mikroreaktor, in dem die Umsetzung mit Sauerstoff erfolgt, und im zweiten Mikroreaktor oder Nachreaktor liegt bei jeweils 2,5 min.

Die Bestimmung von Aldehydumsatz und Selektivität des umgesetzten Aldehyds zu Carbonsäure erfolgt durch gaschromatographische Untersuchungen des Eduktgemisches im By-Pass-Betrieb unter Umgehung des Mikroreaktors sowie durch die gaschromatographische Untersuchung der über die Leitung (9) (einstufige Fahrweise) oder über die Leitung (16) (zweistufige Fahrweise) erhaltenen Rohsäuren im Arbeitsbetrieb. Der Aldehydumsatz ergibt sich dann aus den Aldehydmengen, ermittelt aus den im By-Pass- und Arbeitsbetrieb aufgenommenen Gaschromatogrammen.

Die Carbonsäureselektivität ergibt sich aus den im Arbeitsbetrieb minus By-Pass-Betrieb (bedingt durch im Einsatzaldehyd bereits vorhandener Säurespuren) gaschromatographisch ermittelten Carbonsäuremengen bezogen auf die im Arbeitsbetrieb und im By-Pass-Betrieb gaschromatographisch ermittelten Aldehydmengen.

### Herstellung von n-Pentansäure

### 1. Einstufige Fahrweise (Vergleichsbeispiel)

Die Flüssigphasenoxidation von n-Pentanal zu n-Pentansäure erfolgt gemäß dem Verfahrensschema nach Figur 1 bei einer konstanten Reaktionstemperatur von 80°C bei den in der allgemeinen Beschreibung angegebenen Bedingungen. In der nachfolgenden Tabelle 1 sind die n-Pentanal-Umsätze sowie die Selektvitäten zu n-Pentansäure in Abhängigkeit von dem eingestellten Sauerstoffüberdruck angegeben.

**Tabelle 1: n-Pentanal-Umsätze und Selektivitäten zu n-Pentansäure in Abhängigkeit von dem Sauerstoffüberdruck bei 80°C**

| **Versuchs-Nr.** | **Überdruck [MPa]** | **Umsatz (%) n-Pentanal** | **Selektivität (%) zu n-Pentansäure** |
|---|---|---|---|
| 1 | 0,4 | 82 | 91 |
| 2 | 1,5 | 89 | 85 |
| 3 | 2,0 | 98 | 80 |

Mit steigenden Sauerstoffüberdruck nimmt der n-Pentanalumsatz deutlich zu, gleichzeitig sinkt aber die n-Pentansäureselektivität aufgrund der verstärkten Bildung der Persäure, die sich anschließend unkontrolliert unter Selektivitätsverlust zersetzt. Dennoch werden im Vergleich zu bekannten mittels Mikroreaktoren durchgeführten Aldehydoxidationsreaktionen deutlich höhere Ausbeutewerte an n-Pentansäure beobachtet.

### 2. Zweistufige Fahrweise

Die Flüssigphasenoxidation von n-Pentanal zu n-Pentansäure erfolgt gemäß dem Verfahrensschema nach Figur 2 bei einer konstanten Reaktionstemperatur von 80°C in dem ersten Mikroreaktor, bei einem konstanten Flüssigkeitsdruck von 3,4 MPa in dem zweiten Mikroreaktor (Nachreaktor) und bei den in der allgemeinen Beschreibung angegebenen Bedingungen. In der nachfolgenden Tabelle 2 sind die n-Pentanalumsätze sowie die Selektivitäten zu n-Pentansäure in Abhängigkeit von dem eingestellten Sauerstoffüberdruck und in Abhängigkeit von der Temperatur in dem zweiten Mikroreaktor (Nachreaktor) angegeben.

**Tabelle 2: n-Pentanal-Umsätze und Selektivitäten zu n-Pentansäure in Abhängigkeit von dem Sauerstoffüberdruck und der Temperatur in dem zweiten Mikroreaktor; konstante Temperatur in dem ersten Mikroreaktor bei 80°C und konstanter Flüssigkeitsdruck von 3,4 MPa in dem zweiten Mikroreaktor**

| **Versuchs-Nr.** | **Überdruck [MPa] 1. Mikroreaktor** | **Temperatur (°C) 2. Mikroreaktor** | **Umsatz (%) n-Pentanal** | **Selektivität (%) zu n-Pentansäure** |
|---|---|---|---|---|
| 4 | 0,4 | 30 | 82 | 91 |
| 5 | 0,4 | 70 | 80 | 99 |
| 6 | 0,4 | 80 | 79 | 95 |
| 7 | 1,5 | 80 | 98 | 92 |
| 8 | 2,0 | 80 | Totaloxidation | |

Nach den Versuchen 4 und 5 beobachtet man eine Selektivitätszunahme zu n-Pentansäure bei einer Temperaturerhöhung in dem zweiten Mikroreaktor, so dass man hier von einer gezielten Abreaktion der Persäure ausgehen kann. Versuch 7 zeigt gegenüber der einstufigen Fahrweise nach Versuch 2 verbesserte Umsatz- und Selektivitätswerte. Hier kann vermutet werden, dass in dem Austrag aus dem ersten Mikroreaktor Per-n-pentansäure und der Rest an n-Pentanal in einem molaren Verhältnis von etwa 1:1 vorliegen, so dass es im 2. Mikroreaktor (Nachreaktor) über eine Baeyer-Villiger-Oxidation zur weiteren n-Pentansäurebildung unter Verbrauch von n-Pentanal kommt.

Bei Versuch 8 wird zunächst die Umsetzung in dem 1. Mikroreaktor gemäß den Reaktionsbedingungen nach Beispiel 3 durchgeführt. Bei der sich anschließenden Nachreaktion bei der gleichen Temperatur beobachtet man keine zufriedenstellenden Ergebnisse mehr. Bei dieser Reaktionseinstellung kann man davon ausgehen, dass im Reaktionsaustrag der 1. Reaktionsstufe kaum noch n-Pentanal zugegen ist und neben n-Pentansäure eine vergleichsweise hohe Konzentration an Per-n-pentansäure vorliegt, die sich in unkontrollierter Weise in der Nachreaktionsstufe zersetzt.

### Herstellung von iso-Nonansäure (Vergleichsbeispiel)

Die Flüssigphasenoxidation von iso-Nonanal zu iso-Nonansäure erfolgt gemäß dem Verfahrensschema nach Figur 1 bei einer konstanten Reaktionstemperatur von 80°C bei den in der allgemeinen Beschreibung angegebenen Bedingungen. In der nachfolgenden Tabelle 3 sind die iso-Nonanal-Umsätze sowie die Selektivitäten zu iso-Nonansäure, jeweils bezogen auf das Hauptisomer 3,5,5-Trimethylhexanal bzw. 3,5,5-Trimethylhexansäure in Abhängigkeit von dem eingestellten Sauerstoffüberdruck angegeben.

**Tabelle 3: iso-Nonanal-Umsätze und Selektivitäten zu iso-Nonansäure in Abhängigkeit von dem Sauerstoffüberdruck bei 80°C**

| **Versuchs-Nr.** | **Druck [MPa]** | **Umsatz (%) iso-Nonanal** | **Selektivität (%) zu iso-Nonansäure** |
|---|---|---|---|
| 9 | 0,4 | 64 | 88 |
| 10 | 0,6 | 84 | 86 |
| 11 | 0,8 | 90 | 86 |

Mit steigenden Sauerstoffüberdruck nimmt der iso-Nonanalumsatz deutlich zu, gleichzeitig sinkt aber die iso-Nonansäureselektivität aufgrund der verstärkten Bildung der Persäure, die sich anschließend unkontrolliert unter Selektivitätsverlust zersetzt. Dennoch werden auch bei der iso-Nonanaloxidation im Vergleich zu bekannten mittels Mikroreaktoren durchgeführten Aldehydoxidationsreaktionen deutlich höhere Ausbeuten an der gewünschten Carbonsäure beobachtet.

## Patentansprüche

1. Verfahren zur Herstellung aliphatischer Carbonsäuren mit 3 bis 10 Kohlenstoffatomen durch Oxidation der entsprechenden Aldehyde mit Sauerstoff oder Sauerstoff enthaltenden Gasen, **dadurch gekennzeichnet, dass** die Oxidation in einem Mikroreaktor bei erhöhtem Druck und bei einem Sauerstoffüberschuss, bezogen auf die stöchiometrisch erforderliche Sauerstoffmenge, durchgeführt wird und dass das aus dem Mikroreaktor abgeführte Reaktionsgemisch ohne weitere Sauerstoffzugabe durch mindestens einen Nachreaktor geleitet wird und wobei die Oxidationsreaktion in dem Mikroreaktor unter solchen Bedingungen durchgeführt wird, dass im abgeführten Reaktionsgemisch Aldehyd und gebildete entsprechende Persäure in einem molaren Verhältnis in einem Bereich von 1 : (0,9 bis 1,1), vorzugsweise von 1 : (0,95 bis 1,05), vorliegen.

2. Verfahren nach Anspruch 1, **dadurch gekennzeichnet, dass** die Oxidation bei einer Temperatur von 0 bis 130°C, vorzugsweise von 20 bis 100°C und insbesondere von 40 bis 80°C erfolgt.

3. Verfahren nach Anspruch 1, **dadurch gekennzeichnet, dass** der Mikroreaktor und der unmittelbar folgende Nachreaktor bei der gleichen Temperatur betrieben werden.

4. Verfahren nach Anspruch 1, **dadurch gekennzeichnet, dass** der Mikroreaktor und mindestens ein Nachreaktor bei unterschiedlichen Temperaturen betrieben werden, wobei von Stufe zu Stufe die Temperatur abnimmt und zwischen benachbarten Stufen die Temperaturdifferenz 5 bis 50°C, vorzugsweise 10 bis 20°C, beträgt.

5. Verfahren nach einem oder mehreren der Ansprüche 1 bis 4, **dadurch gekennzeichnet, dass** die Oxidationsreaktion bei einem Druck von 0,4 bis 2,0 MPa und mit dem bis zu Zweifachen der stöchiometrisch erforderlichen Sauerstoffmenge durchgeführt wird,

6. Verfahren nach einem oder mehreren der Ansprüche 1 bis 5, **dadurch gekennzeichnet, dass** das Oxidationsmittel molekularer Sauerstoff ist.

7. Verfahren nach einem oder mehreren der Ansprüche 1 bis 6, **dadurch gekennzeichnet, dass** das Oxidationsmittel ein molekularer Sauerstoff und bis zu 90 Vol-%, insbesondere 30 bis 80 Vol-%, inerte Gase enthaltendes Gasgemisch ist.

8. Verfahren nach Anspruch 7, **dadurch gekennzeichnet, dass** das Gasgemisch Luft ist.

9. Verfahren gemäß einem oder mehreren der Ansprüche 1 bis 8, **dadurch gekennzeichnet, dass** die aliphatische Carbonsäure n-Buttersäure, n-Pentansäure, 2-Methylbuttersäure, 2-Ethylhexansäure, 2-Propylheptansäure oder iso-Nonansäure ist.

10. Verfahren gemäß einem oder mehreren der Ansprüche 1 bis 9, **dadurch gekennzeichnet, dass** der Mikroreaktor eine Mikrokapillare oder ein Mikroblasensäulenreaktor ist.

11. Verfahren gemäß einem oder mehreren der Ansprüche 1 bis 10, **dadurch gekennzeichnet, dass** der Mikroreaktor einen Reaktionsraum mit einen Durchmesser von 1 Millimeter bis 0,01 Millimetern aufweist.

12. Verfahren gemäß einem oder mehreren der Ansprüche 1 bis 11, **dadurch gekennzeichnet, dass** mehrere parallel angeordnete Mikroreaktoren betrieben werden.

13. Verfahren gemäß Anspruch 1, **dadurch gekennzeichnet, dass** mindestens ein Nachreaktor ein Mikroreaktor ist.

## Claims

1. Process for preparing aliphatic carboxylic acids having 3 to 10 carbon atoms by oxidizing the corresponding aldehydes with oxygen or oxygen-containing gases, **characterized in that** the oxidation is performed in a microreactor at elevated pressure and with an oxygen excess based on the stoichiometrically required amount of oxygen, and **in that** the reaction mixture removed from the microreactor is passed through at least one postreactor without further addition of oxygen, and wherein the oxidation reaction in the microreactor is performed under such conditions that aldehyde and corresponding peracid formed are present in the reaction mixture removed in a molar ratio within a range of 1:(0.9 to 1.1), preferably of 1 :(0.95 to 1.05).

2. Process according to Claim 1, **characterized in that** the oxidation is effected at a temperature of 0 to 130°C, preferably of 20 to 100°C and especially of 40 to 80°C.

3. Process according to Claim 1, **characterized in that** the microreactor and the postreactor directly downstream are operated at the same temperature.

4. Process according to Claim 1, **characterized in that** the microreactor and at least one postreactor are operated at different temperatures, the temperature decreasing from stage to stage and the temperature difference between adjacent stages being 5 to 50°C, preferably 10 to 20°C.

5. Process according to one or more of Claims 1 to 4, **characterized in that** the oxidation reaction is performed at a pressure of 0.4 to 2.0 MPa and with up to twice the stoichiometrically required amount of oxygen.

6. Process according to one or more of Claims 1 to 5, **characterized in that** the oxidizing agent is molecular oxygen.

7. Process according to one or more of Claims 1 to 6, **characterized in that** the oxidizing agent is a gas mixture comprising molecular oxygen and up to 90% by volume, especially 30 to 80% by volume, of inert gases.

8. Process according to Claim 7, **characterized in that** the gas mixture is air.

9. Process according to one or more of Claims 1 to 8, **characterized in that** the aliphatic carboxylic acid is n-butyric acid, n-pentanoic acid, 2-methylbutyric acid, 2-ethylhexanoic acid, 2-propylheptanoic acid or isononanoic acid.

10. Process according to one or more of Claims 1 to 9, **characterized in that** the microreactor is a microcapillary or a microscale bubble column reactor.

11. Process according to one or more of Claims 1 to 10, **characterized in that** the microreactor has a reaction space with a diameter of 1 millimeter to 0.01 millimeter.

12. Process according to one or more of Claims 1 to 11, **characterized in that** several microreactors arranged in parallel are operated.

13. Process according to Claim 1, **characterized in that** at least one postreactor is a microreactor.

## Revendications

1. Procédé pour la préparation d'acides carboxyliques aliphatiques comprenant 3 à 10 atomes de carbone par oxydation des aldéhydes correspondants avec de l'oxygène ou des gaz contenant de l'oxygène, **caractérisé en ce que** l'oxydation est réalisée dans un microréacteur à pression augmentée et sous un excès d'oxygène, par rapport à la quantité d'oxygène stoechiométriquement nécessaire, et **en ce que** le mélange réactionnel évacué du microréacteur est guidé sans autre addition d'oxygène au travers d'au moins un post-réacteur, la réaction d'oxydation dans le microréacteur étant réalisée dans des conditions telles que l'aldéhyde et les peracides correspondants formés dans le mélange réactionnel évacué se trouvent dans un rapport molaire dans une plage de 1:(0,9 à 1,1), de préférence de 1:(0,95 à 1,05).

2. Procédé selon la revendication 1, **caractérisé en ce que** l'oxydation a lieu à une température de 0 à 130°C, de préférence de 20 à 100°C et en particulier de 40 à 80°C.

3. Procédé selon la revendication 1, **caractérisé en ce que** le microréacteur et le post-réacteur immédiatement consécutif sont exploités à la même température.

4. Procédé selon la revendication 1, **caractérisé en ce que** le microréacteur et au moins un post-réacteur sont exploités à des températures différentes, la température diminuant d'étage en étage et la différence de température entre des étages adjacents est de 5 à 50°C, de préférence de 10 à 20°C.

5. Procédé selon l'une ou plusieurs des revendications 1 à 4, **caractérisé en ce que** la réaction d'oxydation est réalisée à une pression de 0,4 à 2,0 MPa et avec jusqu'à deux fois la quantité d'oxygène stoechiométriquement nécessaire.

6. Procédé selon l'une ou plusieurs des revendications 1 à 5, **caractérisé en ce que** l'oxydant est l'oxygène moléculaire.

7. Procédé selon l'une ou plusieurs des revendications 1 à 6, **caractérisé en ce que** l'oxydant est un mélange gazeux contenant de l'oxygène moléculaire et jusqu'à 90% en volume, en particulier 30 à 80% en volume de gaz inertes.

8. Procédé selon la revendication 7, **caractérisé en ce que** le mélange gazeux est l'air.

9. Procédé selon l'une ou plusieurs des revendications 1 à 8, **caractérisé en ce que** l'acide carboxylique aliphatique est l'acide n-butyrique, l'acide n-pentanoïque, l'acide 2-méthylbutyrique, l'acide 2-éthylhexanoïque, l'acide 2-propylheptanoïque ou l'acide isononanoïque.

10. Procédé selon l'une ou plusieurs des revendications 1 à 9, **caractérisé en ce que** le microréacteur est un microcapillaire ou un microréacteur à colonne à bulles.

11. Procédé selon l'une ou plusieurs des revendications 1 à 10, **caractérisé en ce que** le microréacteur présente une chambre de réaction présentant un diamètre de 1 millimètre à 0,01 millimètre.

12. Procédé selon l'une ou plusieurs des revendications 1 à 11, **caractérisé en ce qu'**on exploite plusieurs microréacteurs disposés en parallèle.

13. Procédé selon la revendication 1, **caractérisé en ce qu'**au moins un post-réacteur est un microréacteur.
